# EUROPEAN PATENT APPLICATION

(11) **EP 2 526 939 A1**
(43) Date of publication of application: **28.11.2012**
(21) Application number: 11176814.9
(22) Date of filing: 08.08.2011
(51) Int. Cl.: A61K 31/353, A61K 38/01, A61K 36/00, A61P 1/00, A61P 1/04

(54) **Gelatine tannate and associations thereof for use in the treatment of inflammatory gastrointestinal diseases**

(30) Priority: 24.05.2011 IT MI20110934
(71) Applicant: Alonso Cohen, Miguel Angel, 08034 Barcelona (ES)
(72) Inventor: Alonso Cohen, Miguel Angel, 08034 BARCELONA (ES); Di Fulvio, Marco, 6915 PAMBIO-NORANCO (CH)
(74) Representative: Minoja, Fabrizio

(57) **Abstract**

The present invention relates to the use of gelatine tannate, optionally in association with prebiotics or probiotics, for the treatment of inflammatory processes of the gastrointestinal apparatus and of gastrointestinal diseases thereof.

## Description

### Field of the invention

The present invention relates to the use of gelatine tannate, optionally in association with prebiotics or probiotics, for the treatment of inflammatory processes of the gastrointestinal apparatus and of gastrointestinal diseases thereof.

### Background of the invention

Gelatine tannate is a known pharmaceutical agent used to restore the physiological function of the intestinal walls. It is marketed with the trade names of Tasectan® and Tanagel®. It is a complex of animal gelatine and tannic acid which acts mechanically by protecting the intestinal mucosa, thanks to its ability to form a protective, protein-based mucoadhesive film.

Gelatine tannate is able to control and reduce the symptoms associated with diarrhoea resulting from various causes, such as abdominal tension and frequent defecation.

In particular, gelatine tannate is indicated for the treatment of common diarrhoea in children and infants, dysenteriform enterocolitis in children, and exacerbation of intestinal bacteria. From the pharmacological point of view, it is endowed with astringent, anti-diarrhoeic and mucosa protective effects. It does not alter the intestinal flora and it does not have effects on the intestinal motility, making it particularly suitable for administration to children and infants.

Gelatine tannate is unchanged in the acid medium of the stomach, whereas in the alkaline medium of the intestine it undergoes decomposition into tannin and gelatine. The released tannin combines with albuminoidal and mucilaginous substances of the intestinal content, neutralising many products of catarrh in the gastrointestinal walls. Such catarrh products affect the mucosa of these walls and thus maintain the irritation in a vicious circle. In addition, the gelatine acts by providing mechanical protection of gastrointestinal mucosa against the acids and alkaloids produced by bacterial fermentations or by putrefaction during gastrointestinal transit. The pharmaceutical formulation is available in capsule form for adults and as a powder recommended for paediatric use.

### Description of the invention

The inventors of the present invention have now surprisingly found that gelatine tannate, either alone or in association with prebiotics and probiotics, significantly improves pathological conditions associated with gastrointestinal inflammatory processes thanks to simultaneous action on several inflammation mediators, in particular by inhibiting the release of pro-inflammatory cytokines and inducing increased expression of anti-inflammatory cytokines.

Specifically, the inventors of the present application have found that gelatine tannate is able to inhibit *in vitro* the release of pro-inflammatory cytokines, such as, for example, IL-8 and TNF-α, from intestinal epithelial cells stimulated with LPS, while at the same time it does not exert any cytotoxic effect on said cells.

The inventors also found that gelatine tannate, either alone or in association with prebiotics or probiotics, is able to induce *in vivo* the expression of anti-inflammatory cytokines such as, for example, IL-10, while simultaneously reducing pro-inflammatory cytokines such as, for example, IL-6 and TNF-α. Therefore, gelatine tannate is useful in the treatment of ulcerative colitis or other inflammatory diseases of the gastrointestinal district, such as for example Crohn's disease and Irritable Bowel Syndrome (IBS). The compound may also be useful in the treatment of traveller's diarrhoea.

It is therefore an object of the present invention gelatine tannate for use in treating, preventing or ameliorating a symptom of gastrointestinal inflammation in a patient presenting such symptom.

In one embodiment gelatine tannate is a 1:1 mixture by weight ratio of gelatine and tannic acid.

In a preferred embodiment gelatine tannate comprises gelatine obtained from bovine or porcine skin.

In another preferred embodiment gelatine tannate comprises type-A, acid gelatine.

In one embodiment, the gastrointestinal inflammation is chronic gastrointestinal inflammation.

In another embodiment, the gastrointestinal inflammation is acute gastrointestinal inflammation.

In a preferred embodiment, the gastrointestinal inflammation is caused by an inflammatory bowel disease.

In a particularly preferred embodiment, the inflammatory bowel disease is ulcerative colitis.

In another particularly preferred embodiment, the inflammatory bowel disease is Crohn's disease.

In another particularly preferred embodiment, the inflammatory bowel disease is Irritable Bowel Syndrome.

In one embodiment, gelatine tannate is used in association with a probiotic.

In another embodiment, gelatine tannate is used in association with a prebiotic.

In still another embodiment, gelatine tannate is used in association with both a probiotic and a prebiotic.

In a preferred embodiment, the probiotic is selected from strains of Lactobacilli, Bifidobacteria and Enterococci. In a particularly preferred embodiment the probiotic is live Lactobacilli from the *Bacillus clausii* strain.

In another preferred embodiment the prebiotic is selected form mannans, monosaccharides and polysaccharides. In a particularly preferred embodiment the prebiotic is inulin.

Another object of the present invention is a kit containing gelatine tannate and a prebiotic or a probiotic as separate powders for the simultaneous or sequential oral administration.

Another object of the present invention is a kit containing gelatine tannate, a prebiotic and a probiotic as separate powders for the simultaneous or sequential oral administration.

In a preferred embodiment the kit contains gelatine tannate, inulin and live Lactobacilli from the *Bacillus clausii* strain.

The invention is further illustrated by the following examples.

### Example 1 - In vitro evaluation of the anti-inflammatory effects of gelatine tannate either alone or in association with prebiotics and probiotics

In this study, the anti-inflammatory effects of gelatine tannate (TASECTAN-NOVINTETHICAL) on intestinal epithelial cells were examined in an *in vitro* cellular model. Intestinal cells from the Caco-2 cell line were used. These cells present morphological and biochemical characteristics typical of the enterocytes in the intestine and are widely used to study gastrointestinal tract function (Sambuy Y, et al, Cell Biol Toxicol. 2005; 21(1):1-26; Huang Y, et al, Cytokine. 2003; 22(3-4):77-83). The cells were exposed to increasing concentrations of gelatine tannate in the presence or absence of lipopolysaccharide (LPS), an external bacterial cell-wall component capable of inducing an inflammatory response in intestinal cells. The quantity of cytokines secreted by the LPS-stimulated enterocytes was then measured. Cytokines are inflammatory mediators released by epithelial cells when they come into contact with pro-inflammatory substances. They play a fundamental role in regulating the responses to external stimuli such as inflammation or immune challenge. Compromised equilibrium between cytokines with opposing roles is frequently an underlying factor in pathological conditions such as chronic inflammation and defective immune responses. Caco-2 cells exposed to LPS respond with increased production of cytokines, such as IL-8 and TNF-α (Huang Y, et al, Cytokine. 2003; 22(3-4):77-83). Substances with anti-inflammatory activity are capable of reducing the levels of said cytokines (Vanderpool C, et al, Inflamm. Bowel Dis. 2008; 14(11): 1585-96; Roselli M, et al, Br. J. Nutr. 2006;95:1177-1184; Zhang L, et al, J. Nutr. 2005;135:1752-1756).

In the second part of the study, Caco-2 cells were placed in contact with live lactobacilli (*Bacillus clausii*) in order to evaluate the variation in expression of the anti-inflammatory cytokines IL-10 and IL-6 (Smits HH et al, J. Allergy Clin. Immunol. 2005;115:1260 -1267; Von der Weid T et al, Clin. Diagnostic Laborat. Immunol. 8: 695-701)..

Finally, the effect of the simultaneous presence of gelatine tannate in association with *Bacillus clausii* (a probiotic) and inulin (a prebiotic) on LPS-stimulated Caco-2 cells was evaluated.

The following tests were conducted:
- Cytotoxicity tests to exclude any toxic effect of the test substance
- ELISA assays to evaluate anti-inflammatory effects by measuring cytokine production by cells exposed to the pro-inflammatory stimulus.

### Cell cultures

Caco-2 cells were maintained in Minimum Essential Medium (MEM) supplemented with 10% foetal calf serum, 100 U/ml penicillin and 100 µg/ml streptomycin, and incubated at 37°C in a humidified atmosphere containing 95% air and 5% CO₂. The culture medium was replaced every 2-3 days. Twenty-four hours prior to testing, the cells were trypsinized, counted using a haemocytometer and seeded into 96 well plates. The cells were then exposed to increasing concentrations of the test substance, in the presence or absence of 1 µg/ml LPS, for 24 hours.

### Cytotoxicity tests

Cytotoxicity was evaluated using the tetrazolium salt (MTT) metabolisation test.

The cellular viability assay is used to evaluate the toxicity of substances that might compromise the survival of cells they come into contact with. The MTT test is based on the reduction of the yellow tetrazolium salt (3-(4,5-dimethylthiazol-2y1)diphenyltetrazolium bromide) by metabolically active cells to form insoluble blue formazan crystals. In this test, the stained cells are living, and their viability may be quantified using a spectrophotometric microplate reader. The absorbance value obtained is directly proportional to concentration; therefore, the greater the absorbance, the greater the number of viable cells.

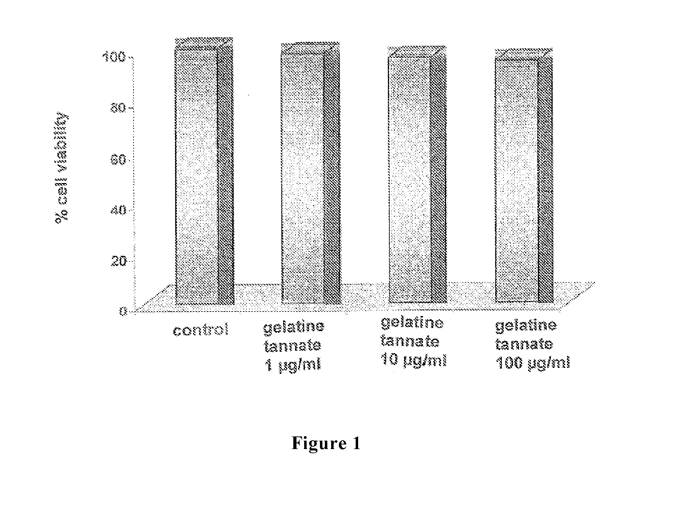

The results are reported in Figure 1 and demonstrate that gelatine tannate is not cytotoxic when left in contact with Caco-2 cells for 24 hours.

### ELISA assays

Cytokines are inflammation mediators released by epithelial cells as a result of the action of irritant or pro-inflammatory substances. IL-8 and TNF-α, in particular, are chemotactic proteins that promote the migration of neutrophils towards damaged tissue, contributing to the progression of inflammation. Interleukin 10 (IL-10) is an anti-inflammatory cytokine produced mainly by monocytes capable of inhibiting the synthesis of pro-inflammatory cytokines, such as IFN-gamma, IL-2 and IL-3. Interleukin 6 (IL-6), secreted mainly by macrophages and T-lymphocytes, has both pro-inflammatory and anti-inflammatory properties; indeed, it is capable of inhibiting TNF-α and promotes the production and action of IL-10.

The cytokines IL-8, TNF-α, IL-10 and IL-6 were determined in the cellfree supernatant collected following treatment of Caco-2 cells by means of an ELISA (Enzyme-Linked ImmunoSorbent Assay) capable of specifically recognising the proteins through the antigen-monoclonal antibody binding. The resulting complex was recognised and bound by an enzyme-conjugated polyclonal antibody. Calculating the enzyme activity, after the addition of substrate, gives a value directly proportional to the quantity of antigen in the test.

The enzyme reaction was blocked using an acidic solution which resulted in the development of a yellow stain. The absorbance was read at λ 450 nm using a spectrophotometric microplate reader.

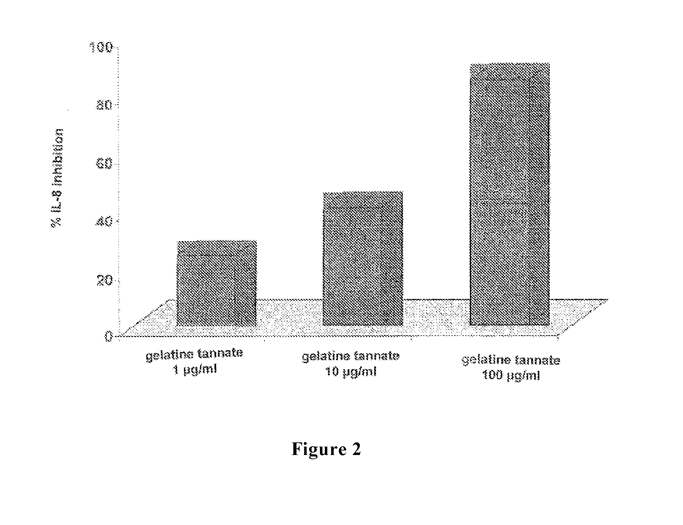

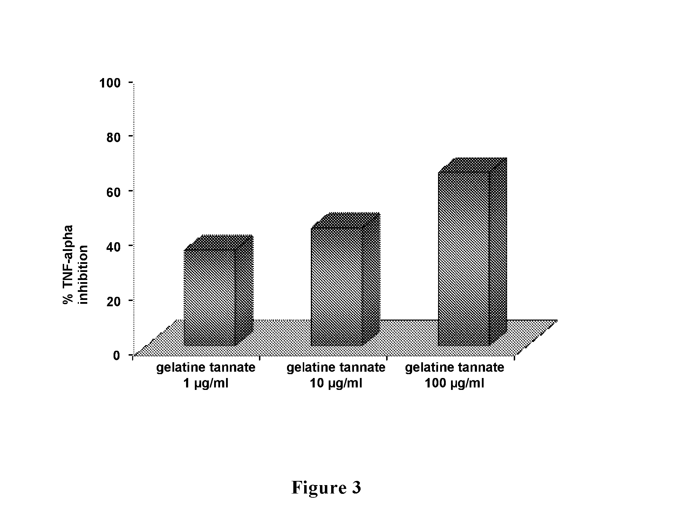

The results reported in Figures 2 and 3 and in Table 1 show that gelatine tannate, when administered alone, inhibited the release of significant quantities of IL-8 and TNF-α from Caco-2 cells stimulated with LPS.

**Table 1. Percentage inhibition of IL-8 and TNF-α expression by various concentrations of gelatine tannate in LPS-stimulated Caco-2 intestinal epithelial cells.**

| | **% IL-8 inhibition** | **% TNF-α inhibition** |
|---|---|---|
| 1 µg/ml gelatine tannate | 24% | 35% |
| 10 µg/ml gelatine tannate | 41% | 43% |
| 100 µg/ml gelatine tannate | 85% | 63% |

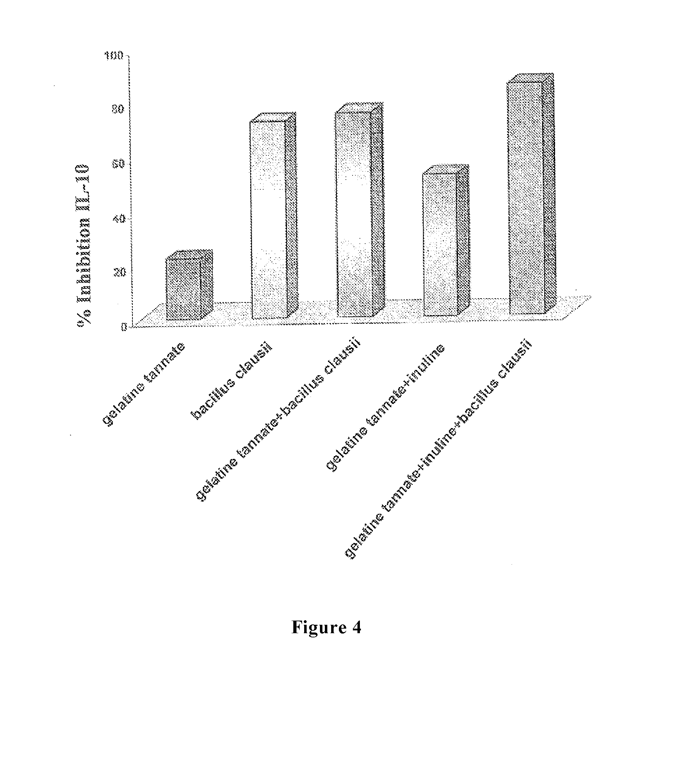

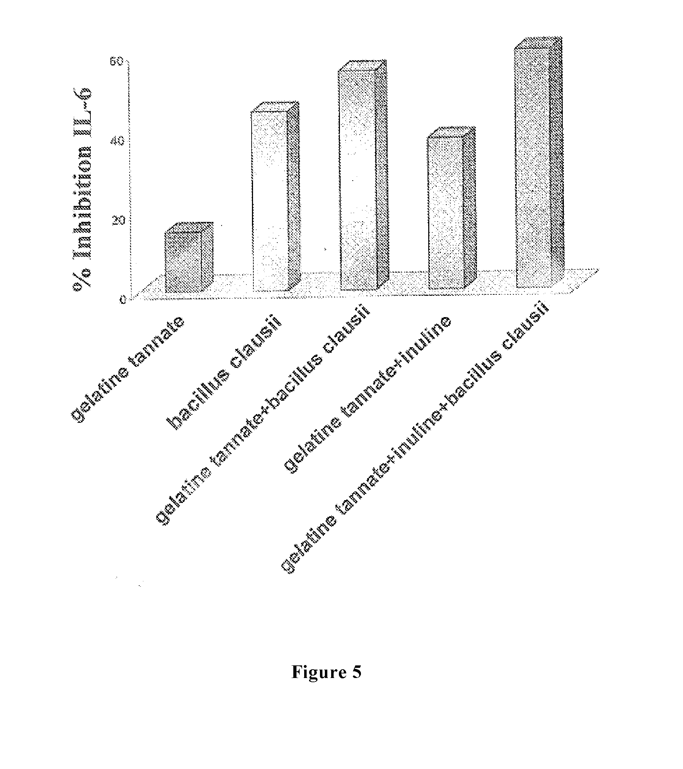

*Bacillus clausii* induced significant quantities of IL-10 and IL-6 in LPS-stimulated Caco-2 cells. Similarly, in association with inulin, gelatine tannate induced significant quantities of IL-10 and IL-6. This release was further increased when cells were treated with gelatine tannate+inulin in association and simultaneously with *Bacillus clausii* (Figures 4 and 5).

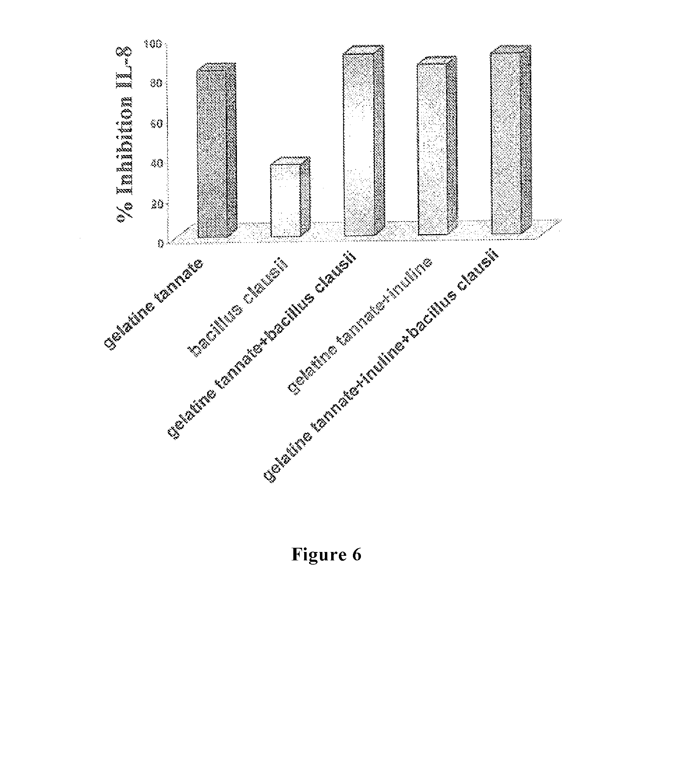

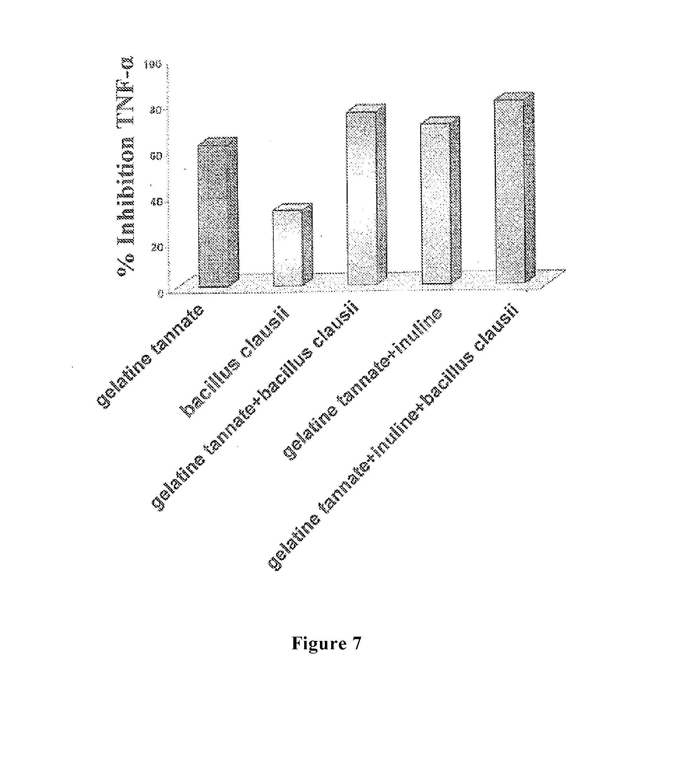

Hence, the association of gelatine tannate and inulin is able of inducing the expression of the anti-inflammatory cytokines IL-10 and IL-6 and significantly reducing the release of pro-inflammatory cytokines, such as IL-8 and TNF-α (Figures 5-7, Table 2).

The results reported in Table 2 and in Figures 4-7 were obtained using the following concentrations:
- *Bacillus clausii:* 20 spores per cell (bacteria/host cell ratio 20/1);
- Gelatine tannate: 100 µg/ml;
- Gelatine tannate + inulin: 100 µg/ml.

The obtained results demonstrate that gelatine tannate is devoid of cytotoxic potential and inhibits important mediators of inflammation in LPS-stimulated intestinal epithelial cells. The results also show that gelatin tannate in association with probiotics and prebiotics can markedly improve pathological conditions associated with gastrointestinal inflammatory processes, thanks to the simultaneous action on several inflammatory mediators, in particular by inhibiting the release of pro-inflammatory cytokines and inducing an increased expression of anti-inflammatory cytokines.

**Table 2. Percentage induction of IL-10 and IL-6 release and inhibition of IL-8 and TNF-α release by gelatine tannate, Bacillus clausii, gelatine tannate + Bacillus clausii in association, gelatine tannate + inulin in association, and gelatine tannate + inulin + Bacillus clausii in association, in LPS-stimulated Caco-2 intestinal epithelial cells**

| | **% IL-10 induction** | **% IL-6 induction** | **% IL-8 inhibition** | **% TNF-α inhibition** |
|---|---|---|---|---|
| Gelatine tannate | 22% | 15% | 83% | 62% |
| *Bacillus clausii* | 72% | 45% | 36% | 33% |
| Gelatine tannate + *Bacillus clausii* | 75% | 55% | 91% | 75% |
| Gelatine tannate + inulin | 52% | 38% | 85% | 70% |
| Gelatine tannate + inulin+ *Bacillus clausii* | 85% | 60% | 90% | 80% |

### Example 2

Tissue levels of IL-6, IL-10 and TNF-α were evaluated in an animal model of experimentally induced colitis (DNBS group) following treatment with either 375 mg inulin (DNBS + inulin group) or with 500 mg gelatine tannate in association with 1.5 g inulin (DNBS + G.T.I. group).

Colitis was induced following the method described by Fornai et al. (J. Pharmacol Exp Ther 2006, 317(3):938-945). Briefly, male adult albino Spargue-Dawley rats weighting 230±20 g received 15 mg of dinitrobenzenesulfonic acid (DNBS) in 0.25 mL of 50% ethanol directly in the lumen of the colon via a PE-60 polyethylene catheter rectally introduced up to a distance of 8 cm from the anal orifice.

Animals were assigned to the following experimental groups:
1. Control group (6 animals): on Day 1 control animals were treated with physiological saline.
2. DNBS group (6 animals): on Day 1 animals received a single administration of DNBS and after 6 days they were scarified.
3. DNBS + inulin treatment group (6 animals): animals received a single administration of DNBS; starting from Day 0 (the day before induction of colitis) and throughout the whole experimental period (6 days), animals of this group were treated daily with 375 mg inulin.
4. DNBS + gelatine tannate + inulin treatment group (G.T.I.) (6 animals): animals received a single administration of DNBS; starting from Day 0 (the day before induction of colitis) and throughout the whole experimental period (6 days), animals of this group were treated daily with 500 mg gelatine tannate and 1.5 g inulin.

At time of sacrifice samples of colon tissue were taken in order to evaluate IL-6, IL-10 and TNF-α using rat-specific ELISA assays. At sacrifice, each colon was completely removed and cryopreserved in isopentane at -80°C until the time of processing through homogenization. Tissues were homogenized in a suitable buffer containing inhibitors of proteases (Apronitin, Leupeptin, Pepstatin, PMSF) using a Potter, then the crude homogenate was centrifuged and the supernatant recovered and subsequently used for the protein and immune-enzymatic assays.

Protein determination were performed using a commercially available solution of Bradford's reagent (Sigma). Samples were diluted as necessary and applied on a well plate, then the Bradford's reagent was added. Reading was performed at 595 nm. The standard plot was obtained using albumin (Sigma).

ELISA assays were performed using commercially available (Searchlight) rat-specific antibodies against IL-6, IL-10 and TNF-α.

Data obtained with the immunoenzymatic assays was normalized to the total protein content of each sample and normalized to the average value of controls.

### IL-6 levels

After 6 days of treatment of the animals with DNBS according to the literature a marked increase of this cytokine was observed (p<0.01 vs. control) in the DNBS group. Treatment with inulin (DNBS + inulin group) did not induce a variation in the IL-6 levels in comparison with the DNBS group. On the other hand, treatment with the gelatine tannate + inulin association (DNBS + G.T.I. group) resulted in a significant reduction of the IL-6 levels in comparison to both the DNBS and the DNBS + inulin groups (p<0.05).

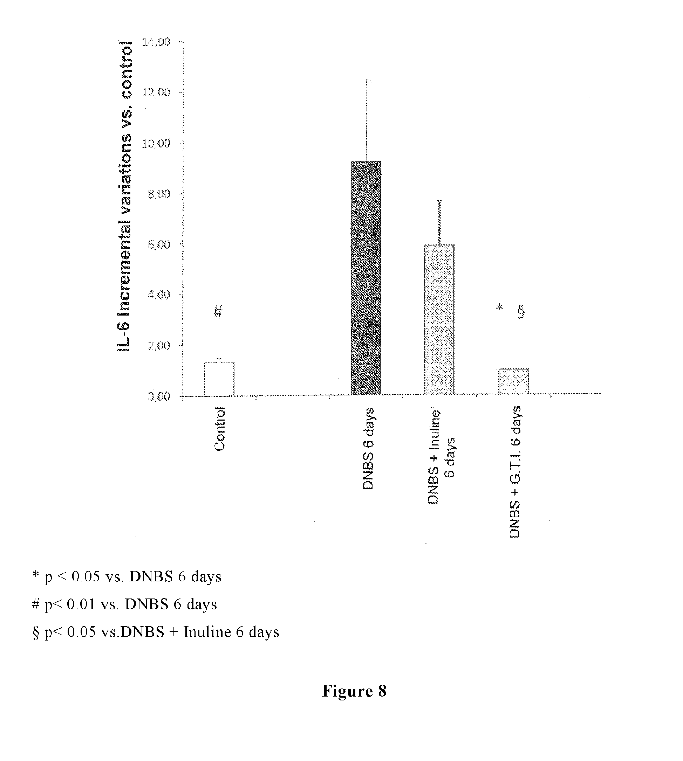

The results are reported in Figure 8.

### IL-10 levels

After 6 days of the treatment of the animals with DNBS a marked increase of IL-10 levels was observed (p<0.05 vs. control) in the DNBS group. Treatment with inulin (DNBS + inulin group) did not induce a variation in the IL-10 levels in comparison with the DNBS group. On the other hand, treatment with the gelatine tannate + inulin association (DNBS + G.T.I. group) resulted in a significant reduction of the IL-10 levels in comparison to the DNBS group (p<0.05).

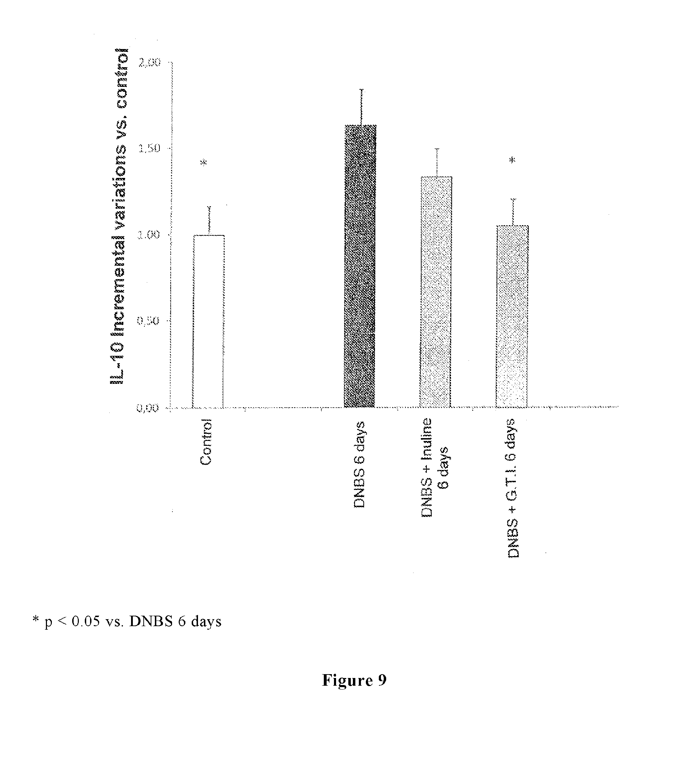

The results are reported in Figure 9.

### TNF-α levels

After 6 days of the treatment of the animals with DNBS a marked increase of this cytokine was observed (p<0.01 vs. control) in the DNBS group. Treatment with inulin (DNBS + inulin group) did not induce statistically significant variation in the TNF-α levels in comparison with the DNBS group. On the other hand, treatment with the gelatine tannate + inulin association resulted in a significant reduction of the TNF-α levels in comparison to both the DNBS (p<0.01) and the DNBS + inulin (p<0.05) groups.

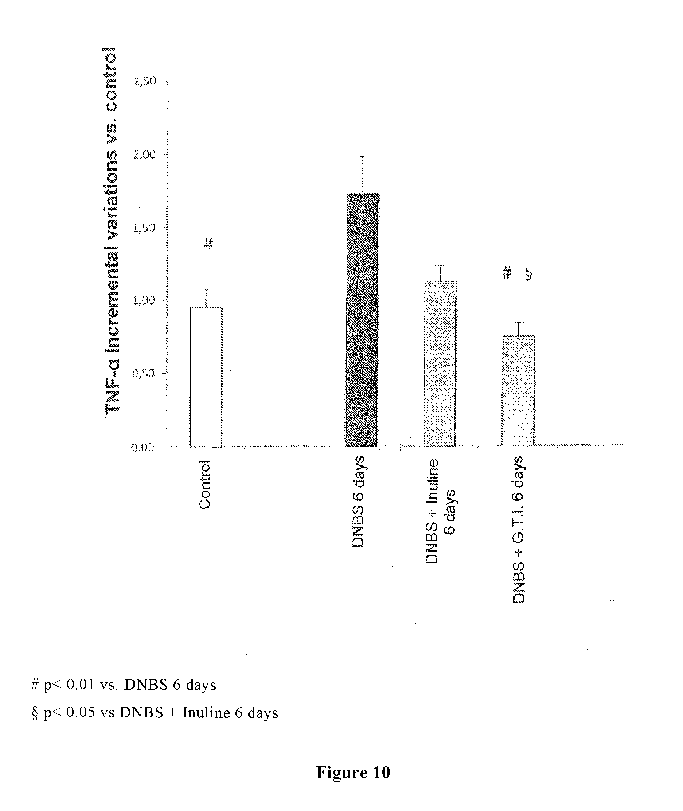

The results are reported in Figure 10.

The above mentioned determinations of IL-6, IL-10 and TNF-α, expressed as average and standard deviation of the increments, are reported in Table 3.

**Table 3**

| | **Average** | | | | **Standard Deviation** | | |
|---|---|---|---|---|---|---|---|
| | **IL-6** | **IL-10** | **TNF-α** | | **IL6** | **IL10** | **TNFa** |
| Control | 1.34 | 1.00 | 0.95 | | 0.145 | 0.163 | 0.116 |
| DNBS (Day 6) | 9.22 | 1.63 | 1.72 | | 3.237 | 0.213 | 0.258 |
| DNBS + Inulin (Day 6) | 5.88 | 1.32 | 1.12 | | 1.777 | 0.169 | 0.112 |
| DNBS + G.T.I. (Day 6) | 1.00 | 1.05 | 0.75 | | 0.000 | 0.152 | 0.90 |

The above data demonstrate that in the acute treatment of ulcerative colitis the association of gelatine tannate + inulin is able to induce a reduction of the inflammatory phase, decreasing the release of the IL-6 and TNF-α inflammatory mediators while simultaneously increasing that of anti-inflammatory molecules, such as IL-10.

### Example 3 - Drug Product Formula

Gelatine tannate is the active principle in Tanagel® Powder and it is composed by tannic acid and gelatine.

Gelatine used in the manufacture of gelatine tannate is type A acid gelatine whose dissolution pH is 5.5, with a capacity of gelification of 40/60 grades bloom (Viscosity of 27 mps)

Gelatine is a pure and natural protein, easily digested, without any content of cholesterol and without any odour or flavour.

The content of gelatine is 84-90% of collagen protein and water. The protein is composed by 18 essential amino acids.

Gelatine is classified as a food product that from an energetic point of view is hypo caloric (dissolution of 0.5 L = 40 Kcal).

| Gelatine formula: | |
|---|---|
| Glycine | 25.5% |
| Alanine | 8.7% |
| Valine | 2.5% |
| Leucine | 3.2% |
| Isoleucine | 1.4% |
| Cystine and Cysteine | 0.1% |
| Methionine | 1% |
| Phenylalanine | 2.2% |
| Proline | 18% |
| Hydroxyproline | 14% |
| Serine | 0.4% |
| Threonine | 1.9% |
| Tyrosine | 0.5% |
| Aspartic Acid | 6.6% |
| Glutamic Acid | 11.4% |
| Arginine | 8.1% |
| Lysine | 4.1% |
| Histidine | 0.8% |

## Claims

1. Gelatine tannate for use in treating, preventing or ameliorating a symptom of gastrointestinal inflammation in a patient presenting such symptom.

2. Gelatine tannate according to claims 1, comprising gelatine obtained from bovine or porcine skin.

3. Gelatine tannate according to claims 1 or 2, comprising type-A, acid gelatine.

4. Gelatine tannate according to claim 1, wherein the treating, preventing or ameliorating of a symptom of gastrointestinal inflammation comprise inhibiting the release of pro-inflammatory cytokines selected from IL-8 and TNF-α, and/or inducing the increased expression of anti-inflammatory cytokines selected from IL-10 and IL-6.

5. Gelatine tannate according to claim 1, wherein the gastrointestinal inflammation is chronic or acute gastrointestinal inflammation.

6. Gelatine tannate according to claim 1, wherein the gastrointestinal inflammation is caused by inflammatory bowel disease.

7. Gelatine tannate according to claim 6, wherein the inflammatory bowel disease is ulcerative colitis, Chron's disease or Irritable Bowel Syndrome.

8. Gelatine tannate according to claim 1, in association with a probiotic or a prebiotic.

9. Gelatine tannate according to claim 1, in association with both a probiotic and a prebiotic.

10. Gelatine tannate according to claim 8 or 9, wherein the probiotic is selected from Lactobacilli, Bifidobacteria and Enterococci.

11. Gelatine tannate according to claim 10, wherein the probiotic is live lactic acid bacteria of the strain of *Bacillus clausii.*

12. Gelatine tannate according to claim 8 or 9, wherein the prebiotic is selected from mannans, monosaccharides and polysaccharides.

13. Gelatine tannate according to claim 12, wherein the prebiotic is inuline.

14. Kit of parts containing gelatine tannate and a prebiotic or a probiotic as separate powders for the simultaneous or sequential oral administration.

15. Kit of parts containing gelatine tannate, a prebiotic and a probiotic as separate powders for the simultaneous or sequential oral administration.

16. The kit of claim 15, containing gelatine tannate, inuline and live lactic acid bacteria of the strain of *Bacillus clausii.*
